# EUROPEAN PATENT SPECIFICATION

(11) **EP 2 642 251 B1**
(45) Date of publication and mention of the grant of the patent: **09.08.2017**
(21) Application number: 13159923.5
(22) Date of filing: 19.03.2013
(51) Int. Cl.: G01C 21/20, G01C 22/00, G06F 19/00, G06F 17/50

(54) **Required time calculating system, required time calculating method, and computer-readable recording medium storing required time calculating program**
System zur Berechnung des Zeitbedarfs, Verfahren zur Berechnung des Zeitbedarfs und computerlesbares Aufzeichnungsmedium zur Speicherung des Programms zur Berechnung des Zeitbedarfs
Système de calcul de temps requis, procédé de calcul de temps requis et support d'enregistrement lisible par ordinateur stockant un programme de calcul de temps requis

(30) Priority: 19.03.2012 JP 2012061784
(43) Date of publication of application: 25.09.2013
(73) Proprietor: CASIO COMPUTER CO., LTD., Shibuya-ku, Tokyo 151-8543 (JP)
(72) Inventor: Ura, Kazuo, Hamura-shi, Tokyo 205-8555 (JP); Onumata, Yuichi, Hamura-shi, Tokyo 205-8555 (JP)
(74) Representative: Grünecker Patent- und Rechtsanwälte PartG mbB

(56) References cited:
- WO-A2-2006/065679
- JP-A- H09 178 869
- US-A1- 2008 103 794
- US-A1- 2009 319 172

## Description

### BACKGROUND OF THE INVENTION

### 1. Field of the Invention

The present invention relates to a required time calculating system, a required time calculating program, and a required time calculating method. Specifically, the present invention relates to a required time calculating system, a required time calculating program, and a required time calculating method by which assistance, such as a simulation of race time and instructions regarding running pace during a race, can be easily and accurately provided to a participant of a competitive sport such as a marathon or cycling.

### 2. Description of the Related Art

US 2008/103794 A1 relates to a virtual scenario generator that applies a virtual scenario to real-world data. US 2009/319172 relates to a travel time prediction system.

In recent years, because of rising health consciousness, more and more people are performing daily exercises, such as running, walking, and cycling, to maintain their wellness or improve their health condition. In addition, an increasing number of people are aiming to participate in competitions, such as marathon events and cycling races, through these daily exercises. Among these people aiming to participate in competitions, there is a rising demand for efficient and effective training methods that enable them to achieve good results in the competitions.

Conventionally, as a method for keeping track of a body condition during exercise, a method is known in which heart rate, the number of walking steps, a travel distance, burned calories, and the like are measured and recorded by various sensors, such as a heart rate monitor, a pedometer, a Global Positioning System (GPS) receiver, and the like being attached, as described in Japanese Patent Application Laid-Open (Kokai) Publication No. 2010-264246.

People aiming to participate in competitions such as marathons have a strong interest in knowing their own athletic abilities, such as their stamina, in addition to various data for tracking their body conditions. They are interested in knowing the results they may achieve in an actual competition, or the kind of training required to enhance their athletic abilities to achieve their desired results in the competition.

As a simulation method for competitions such as this, a method for marathons and the like is known in which travel time per arbitrary unit distance serving as one's reference is measured and the travel time is multiplied by a coefficient of exercise intensity. In addition, a method is known in which a calculation chart is used that is based on VDOT proposed by exercise physiology professor, Jack Daniels.

Moreover, a method in which training guidance is provided based on past exercise history (body condition and travel time), and a method in which various information are provided in real-time during a competition by a target result being set in advance are known, as described in Japanese Patent Application (Kohyo) Publication No. 2008-524589 (also published as WO 2006/065679).

As just described, in the simulation methods for competitions which are different from daily exercise and training, predicted completion time for, for example, a marathon can be calculated based on data regarding past travel times and athletic abilities. However, in these simulation methods, the features of an actual marathon course or athletic abilities when running the actual course are not sufficiently taken into consideration. Accordingly, there is a problem in that the accuracy and reliability of predicted time is low.

In addition, in Japanese Patent Application (Kohyo) Publication No. 2008-524589, only information related to a body condition and geographic information are provided as advice and instructions regarding how to run to achieve target time during a competition such as a marathon, which are insufficient for use as advice on achieving a target result.

Accordingly, there is demand among people aiming to participate in competitions for a system or a method by which data regarding travel times and athletic abilities acquired through daily exercise and training are effectively used and suitable assistance for achieving a target result in a competition is provided.

The present invention has been conceived in light of the above-described problem, and an object of the present invention is to provide a required time calculating system, a required time calculating program, and a required time calculating method by which, based on the amount of time taken to travel a certain route such as a daily training course, the amount of time required to travel a different route such as a competition course is calculated.

### SUMMARY OF THE INVENTION

In accordance with one aspect of the present invention, there is provided a required time calculating system according to claim 1.

In accordance with another aspect of the present invention, there is provided a non-transitory computer-readable storage medium having stored thereon a required time calculating program that is executable by a computer, the program being executable by the computer to perform functions according to claim 10. In accordance with another aspect of the present invention, there is provided a required time calculating method according to claim 11. Further advantageous features are defined in the dependent claims.

In the present invention, based on the amount of time taken to travel a certain route, the amount of time required to travel a different route can be calculated.

The above and further objects and novel features of the present invention will more fully appear from the following detailed description when the same is read in conjunction with the accompanying drawings. It is to be expressly understood, however, that the drawings are for the purpose of illustration only and are not intended as a definition of the limits of the invention.

### BRIEF DESCRIPTION OF THE DRAWINGS

FIG. 1A and FIG. 1B are structural diagrams outlining a required time calculating system according to an embodiment of the present invention;
FIG. 2 is a block diagram showing an example of the structure of a chest sensor adopted in the required time calculating system according to the embodiment;
FIG. 3 is a block diagram showing an example of the structure of a wrist coordinator adopted in the required time calculating system according to the embodiment;
FIG. 4 is a block diagram showing an example of the structure of an information terminal adopted in the required time calculating system according to the embodiment;
FIG. 5 is a flowchart of a database constructing method in a required time calculating method according to the embodiment;
FIG. 6A and FIG. 6B are schematic diagrams showing examples of training data collected in the database constructing method according to the embodiment;
FIG. 7 is a flowchart of a simulation method in the required time calculating method according to the embodiment;
FIG. 8A and FIG. 8B are schematic diagrams showing an example of a competition course and topographic data thereof in the simulation method according to the embodiment;
FIG. 9A to FIG. 9C are conceptual diagrams showing a method for calculating predicted time in the simulation method according to the embodiment;
FIG. 10 is a diagram showing an example of exercise assistance information generated in the simulation method according to the embodiment;
FIG. 11 is a flowchart of the required time calculating method according to the embodiment;
FIG. 12A to FIG. 12I are schematic diagrams showing display examples of exercise assistance information provided to a user during a competition by the required time calculating method according to the embodiment;
FIG. 13A to FIG. 13C are structural diagrams outlining a variation example of the required time calculating system according to the embodiment of the present invention;

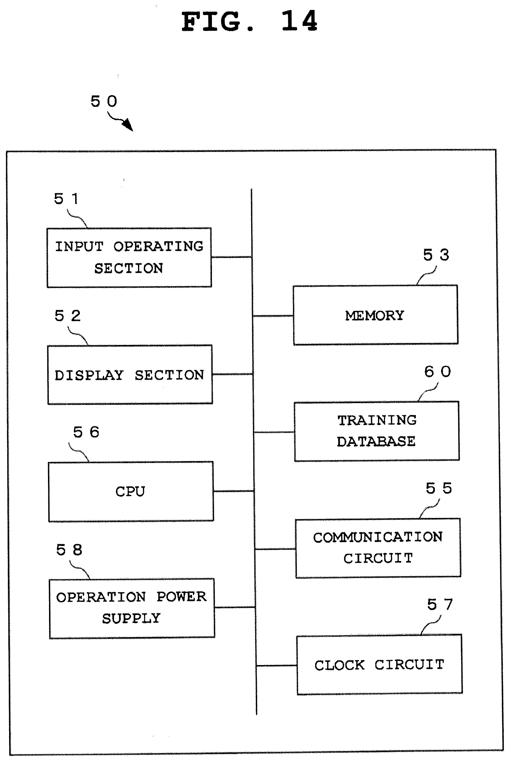

FIG. 14 is a block diagram showing an example of the structure of a server adopted in the required time calculating system according to the embodiment; and
FIG. 15A to FIG. 15C are structural diagrams outlining a second variation example of the required time calculating system according to the embodiment of the present invention.

### DETAILED DESCRIPTION OF THE PREFERRED EMBODIMENTS

In the explanation below, an instance is described in which a user is participating in a marathon event that is an example of a competition.

### (Required Time Calculating System)

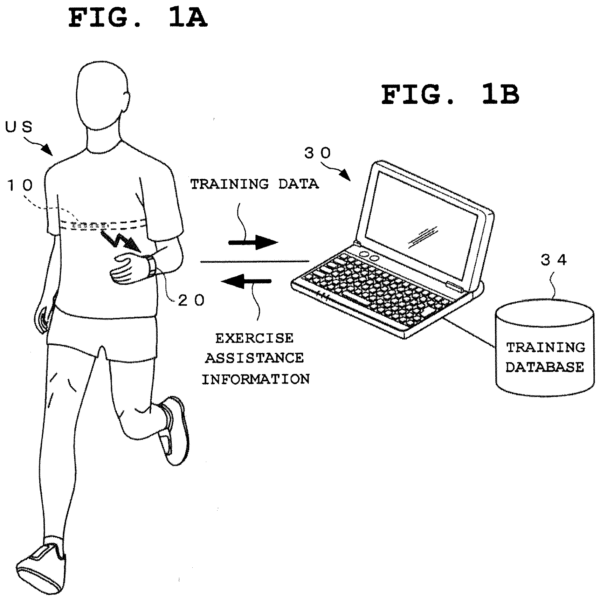

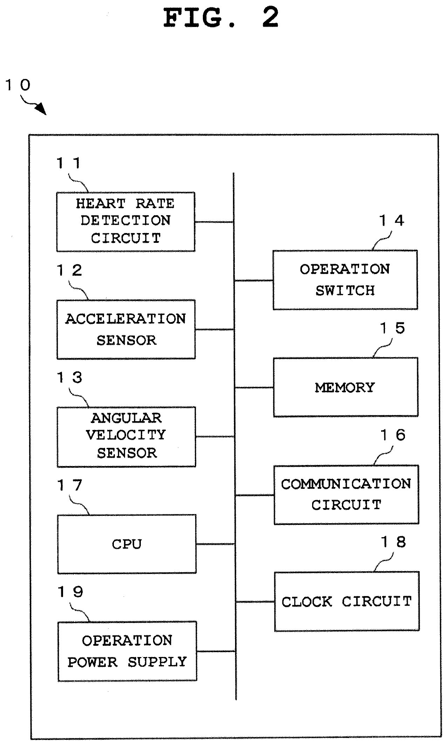

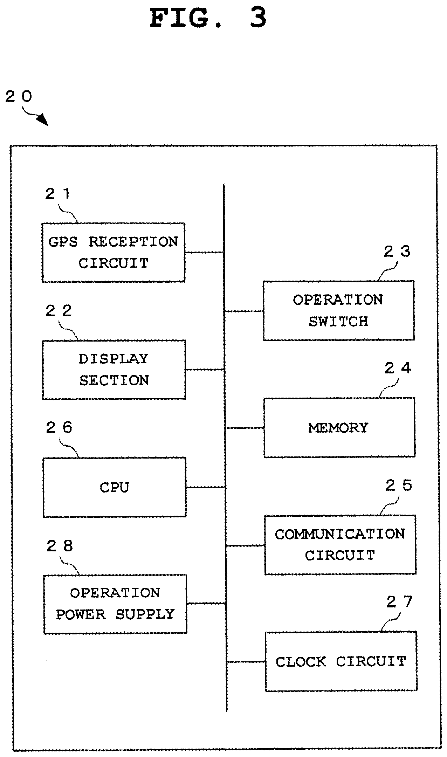

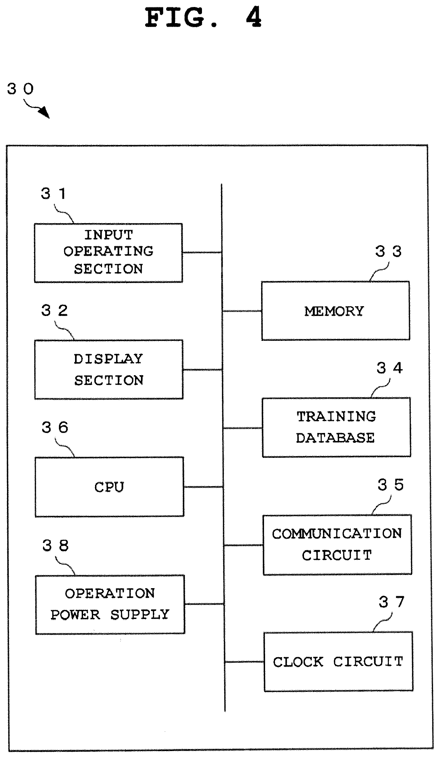

FIG. 1A and FIG. 1B are structural diagrams outlining a required time calculating system according to an embodiment of the present invention, and FIG. 2 is a block diagram showing an example of the structure of a chest sensor adopted in the required time calculating system according to the present embodiment. Also, FIG. 3 is a block diagram showing an example of the structure of a wrist coordinator adopted in the required time calculating system according to the present embodiment, and FIG. 4 is a block diagram showing an example of the structure of an information terminal adopted in the required time calculating system according to the present embodiment.

As shown in FIG. 1A and FIG. 1B, the required time calculating system according to the present embodiment includes a chest sensor 10 and a wrist coordinator 20 that are attached to a user US, and an information terminal 30.

The chest sensor 10 is a chest-attached type sensor that detects at least the biological information, such as heart rate, and the exercise condition of the user US during exercise. This chest sensor 10 is attached by the belt thereof being wrapped around the chest of the user US, as shown in FIG. 1A. Specifically, the chest sensor 10 includes a heart rate detection circuit 11, an acceleration sensor (second sensor) 12, an angular velocity sensor (gyro sensor; third sensor) 13, an operation switch 14, a memory 15, a communication circuit 16, a Central Processing Unit (CPU) 17, a clock circuit 18, and an operation power supply 19, as shown in FIG. 2.

The heart rate detection circuit 11 is provided, for example, on the inner surface side of a belt member that is used to attach the chest sensor 10 to the chest of the user US, and connected to a pair of electrodes (not shown) arranged to come in direct contact with the chest of the user US. This heart rate detection circuit 11 detects heart rate based on changes in electrocardiographic signals outputted from the electrodes. The detected heart rate is stored in the heart rate data storage area of the memory 15.

The acceleration sensor 12 measures the rate of change in movement speed (acceleration) during exercise by the user US, and the angular velocity sensor 13 measures change in a movement direction during exercise by the user US. The acceleration data measured by the acceleration sensor 12 and the angular velocity data measured by the angular velocity sensor 13 are associated with heart rate data detected by the heart rate detection circuit 11, and stored in the acceleration data storage area and the angular velocity data storage area of the memory 15 , respectively. Note that, when the reception status of radio waves from a GPS satellite is poor or the radio waves cannot be received by a GPS reception circuit (first sensor) 21 provided in the wrist coordinator 20 described hereafter, these acceleration data and angular velocity data are used as supplementary data for detecting the position of the user US. Therefore, the acceleration sensor 12 and the angular velocity sensor 13 should preferably be attached to the trunk of the upper body to allow the movement speed and the movement direction of the user US to be more accurately detected.

The operation switch 14 includes at least a power supply switch and controls the power-ON and power-OFF of the chest sensor 10 by supplying power supply voltage supplied from the operation power supply 19 to each component or interrupting the supply. The memory 15 mainly has a non-volatile memory that stores heart rate data detected by the heart rate detection circuit 11, acceleration data measured by the acceleration sensor 12, and angular velocity data measured by the angular velocity sensor 13 in association with one another. Note that the memory 15 may include a Read-Only Memory (ROM) that stores control programs for performing various functions of the heart rate detection circuit 11, the memory 15, and the communication circuit 16. The CPU 17 actualizes the functions of the heart rate detection circuit 11, the acceleration sensor 12, the angular velocity sensor 13, the memory 15, and the communication circuit 16 by performing processing in accordance with these control programs. Also note that the control programs may be loaded into the CPU 17 in advance, and the non-volatile memory section constituting the memory 15 may include a removable storage medium such as a memory card which can be detachably attached to the chest sensor 10.

The communication circuit 16 functions as an interface when the current heart rate data detected by the heart rate detection circuit 11, the current acceleration data measured by the acceleration sensor 12, and the current angular velocity data measured by the angular velocity sensor 13, or the heart rate data, the acceleration data, and the angular velocity data stored in the memory 15 are transmitted to the wrist coordinator 20. Here, for example, various wireless communication methods and wired communication methods using communication cables can be used as a method for transmitting heart rate data, acceleration data, and angular velocity data to the wrist coordinator 20 via the communication circuit 16.

In the case where the above-described data is transmitted by a wireless communication method, Bluetooth (registered trademark) Low Energy (LE) established as a low power-consumption type technology can be favorably adopted, among the specifications of Bluetooth (registered trademark) that is a short-range radio technology. As in the case of typical Bluetooth (registered trademark), Bluetooth (registered trademark) LE is a radio technology using low-power radio waves, which uses a 2. 4GHz band frequency and does not require licensing. A characteristic of Bluetooth (registered trademark) LE is that data can be transmitted using very little power, and therefore data transmission can be favorably performed even with low power generated by a coin-type battery, a button-type battery, or an energy harvesting technology that has been receiving attention in recent years.

The CPU 17 runs a predetermined control program based on a basic clock generated in the clock circuit 18 and thereby controls the respective operations of the heart rate detection circuit 11, the acceleration sensor 12, the angular velocity sensor 13, the memory 15, and the communication circuit 16. The clock circuit 18 generates a basic clock based on synchronization signals transmitted from the wrist coordinator 20 , and generates an operation clock that regulates operation timing for each configuration of the chest sensor 10 based on the basic clock. As a result, synchronization of time data is achieved between the chest sensor 10 and the wrist coordinator 20. This synchronization operation is performed constantly or at time intervals between the chest sensor 10 and the wrist coordinator 20. Also, the clock circuit 18 clocks acquisition timing for heart rate data, acceleration data, and angular velocity data, and outputs it as time data. This time data is associated with the heart rate data, the acceleration data, and the angular velocity data, and stored in a predetermined storage area of the memory 15.

As the operation power supply 19, power supply by an energy harvesting technology that generates power using energy such as vibrations, light, heat, and electromagnetic waves, and the like can be used, in addition to the above-described coin-type battery and button-type battery. Moreover, a secondary battery, such as a lithium ion battery, may also be used as the operation power supply 19.

The wrist coordinator 20 is a wrist-attached type or wristband-type sensor that detects at least the position of the user US during exercise, and displays predetermined information and data. This wrist coordinator 20 is attached by the band thereof being wrapped around the wrist of the user US, as shown in FIG. 1A. Specifically, the wrist coordinator 20 includes the GPS reception circuit 21, a display section 22, operation switches 23, a memory 24, a communication circuit 25, a CPU 26, a clock circuit 27, and an operation power supply 28, as shown in FIG. 3.

The GPS reception circuit 21 receives radio waves from a plurality of GPS satellites, and thereby detects a geographical position (positional information) composed of latitude and longitude, and the altitude (elevation) of this position (altitude information). Also, the GPS reception circuit 21 detects the movement speed (running speed) of the user US using the Doppler shift effect of radio waves from the GPS satellites. GPS data composed of the detected position and altitude (topographic data), the movement speed, and the like are stored, for example, in the GPS data storage area of the memory 24.

The display section 22 has a display panel such as a liquid crystal display panel, and displays on the information terminal 30 various exercise assistance information (described in detail hereafter) generated by simulation processing described hereafter, at least during a competition such as a marathon. This display section 22 may be configured to display time information, GPS data, heart rate data transmitted from the chest sensor 10, or various information calculated based on acceleration data and angular velocity data, such as burned calories calculated from the acceleration data and weight data of the user US, during the above-described competition or training. These pieces of information may be simultaneously displayed on the display panel, or may be successively displayed by the operation of the operation switches 23.

The operation switches 23 include at least a mode switching switch and a display switching switch. By the operation of the mode switching switch, the current mode is controlled to be switched to, for example, an exercise assistance information display mode, a training data display mode, or a menu display mode. In the exercise assistance information display mode, various exercise assistance information generated by the simulation processing described hereafter are displayed. In the training data display mode, heart rate data and GPS data acquired by the chest sensor 10 and the wrist coordinator 20, training data such as burned calories, time information such as that of a stop watch, and the like are displayed. In the menu display mode, a menu screen for performing various setting operations (such as time adjustment and the selection of a communication method) for the wrist coordinator 20 is displayed. Also, by the operation of the display switching switch, screen display in each mode is switched.

Note that the display panel of the display section 22 may be constituted by a touch panel. In this instance, display similar to those described above is actualized by a touch operation on the display panel (touch panel), in addition to or in place of using the mode switching switch and the display switching switch.

The memory 24 includes a non-volatile memory, and stores heart rate data, acceleration data, and angular velocity data transmitted from the chest sensor 10 and GPS data detected by the GPS reception circuit 21, in association with one another. In addition, in the non-volatile memory section of the memory 24, various exercise assistance information generated by the simulation processing described hereafter are stored. Note that the memory 24 may also include a Read-Only Memory (ROM) that stores control programs (software) for performing various functions of the GPS reception circuit 21, the display section 22, the memory 24, and the communication circuit 25. The CPU 26 actualizes the functions of the GPS reception circuit 21, the display section 22, the memory 24, and the communication circuit 25 by performing processing in accordance with these control programs. Also note that the control programs may be loaded into the CPU 26 in advance, and the non-volatile memory section constituting the memory 24 may include a removable storage medium such as a memory card which can be detachably attached to the wrist coordinator 20.

The communication circuit 25 functions as an interface when various training data and exercise assistance information are transmitted between the wrist coordinator 20 and the chest sensor 10 and between the wrist coordinator 20 and the information terminal 30. This communication circuit 25 receives heart rate data, acceleration data, and angular velocity data from the chest sensor 10 and transmits synchronization signals based on a basic clock generated in the clock circuit 27 to the chest senor 10 using the above-described wireless communication method or wired communication method. Also, the communication circuit 25 transmits, to the information terminal 30, heart rate data, acceleration data, and angular velocity data transmitted from the chest sensor 10 and stored in the memory 24, and GPS data detected by the GPS reception circuit 21 and stored in the memory 24. Moreover, the communication circuit 25 receives exercise assistance information transmitted from the information terminal 30. Note that various wireless and wired communication methods, a data transfer method using a memory card, and the like can be used as the method for transmitting various training data such as heart rate data, acceleration data, angular velocity data, and GPS data, and various exercise assistance information between the wrist coordinator 20 and the information terminal 30 via the communication circuit 25.

In the case where a wireless communication method is used to transmit various training data and various exercise assistance information between the wrist coordinator 20 and the information terminal 30, for example, communication using Bluetooth or infrared communication can be favorably used. Also, in the case where a wired communication method is used, the wrist coordinator 20 and the information terminal 30 may be directly connected by a communication cable, or the wrist coordinator 20 may be attached to or placed on a data transfer dock or pad connected to the information terminal 30 by a cable. Note that the data transfer dock or pad herein may adopt a contact-type data transfer method in which the dock or pad is electrically connected with the wrist coordinator 20 by its electrodes coming in direct contact with those of the wrist coordinator 20. Alternatively, it may adopt a non-contact-type data transfer method in which the dock or pad is electrically connected with the wrist coordinator 20 without its electrodes coming in direct contact with those of the wrist coordinator 20. In the case where a wired communication method is used, the operation power supply 28 of the wrist coordinator 20 should preferably be charged by the wrist coordinator 20 being connected to the information terminal 30 via a communication cable or the data transfer dock or pad, and power being supplied from the information terminal 30.

The CPU 26 runs a predetermined control program based on a basic clock generated in the clock circuit 27 and thereby controls the respective operations of the GPS reception circuit 21, the display section 22, the memory 24, and the communication circuit 25. The clock circuit 27, which includes an oscillator for generating a basic clock, generates an operation clock that regulates the operation timing of each component of the wrist coordinator 20, and synchronization signals for the synchronization of time data with the chest sensor 10, based on the basic clock. Also, the clock circuit 27 clocks the acquisition timing of GPS data, and outputs it as time data. The time data is stored in a predetermined storage area of the memory 24 in association with time data associated with heart rate data, acceleration data, and angular velocity data.

As the operation power supply 28, a coin-type battery, a button-type battery, the above-described power supply by an energy harvesting technology, or a secondary battery such as a lithium ion battery can be used. In the case of the above-described configuration where the operation power supply 28 of the wrist coordinator 20 is charged by the wrist coordinator 20 being connected to the information terminal 30, the secondary battery is used as the operation power supply 28.

The information terminal 30 is, for example, a laptop-type, desktop-type, or tablet-type personal computer, as shown in FIG. 1B, and includes an input operating section 31 and a display section 32 so that the user US can operates it and view information. More specifically, the information terminal 30 includes the input operating section 31, the display section 32, a memory 33, a training database 34, a communication circuit 35, a CPU 36 (required time calculating section), a clock circuit 37, and an operation power supply 38, as shown in FIG. 4. Note that the training database 34 may be included in the information terminal 30 as shown in FIG. 4, or may be provided outside of the information terminal 30 and connected thereto by a connection cable, as shown in FIG. 1B.

The input operating section 31 includes an input device, such as a keyboard, a mouse, a touch pad, or a touch panel. By the operation of the input device by the user US, an arbitrary icon or menu on a screen displayed on the display section 32 is selected, or arbitrary information is inputted. The display section 32 includes a display panel such as a liquid crystal display panel, or a monitor, and displays various training data acquired by the chest sensor 10 and the wrist coordinator 20, and various exercise assistance information generated by the simulation processing using the training database 34 where the training data have been stored, in a predetermined format such as numerical values, graphs, and column headings. The details of these various training data and exercise assistance information that are displayed on the display section 32 will be described later.

The memory 33 mainly includes a training data memory that stores heart rate data, acceleration data, angular velocity data, and GPS data transmitted from the wrist coordinator 20 in association with one another, an exercise assistance information memory that stores various exercise assistance information generated in the simulation processing using the training database 34, and a program memory that stores control programs (software) for performing various functions of the display section 32, the memory 33, the training database 34, and the communication circuit 35. The CPU 36 actualizes the functions of the display section 32, the memory 33, the training database 34, and the communication circuit 35 by performing processing in accordance with these control programs.

The training database 34 stores various training data acquired by the chest sensor 10 and the wrist coordinator 20 during the user's training performed before a competition such as a marathon event, in a predetermined storage format. From a number of training data stored in this training database 34, training data corresponding to the topography of a competition course (calculated route) is extracted during a simulation of the competition described hereafter. The details of the training database 34 will be described later.

The communication circuit 35 functions as an interface when various training data and exercise assistance information generated by the simulation processing are transmitted between the information terminal 30 and the wrist coordinator 20. This communication circuit 35 receives various training data such as heart rate data, acceleration data, angular velocity data, and GPS data from the wrist coordinator 20, using the above-described wireless communication method, wired communication method, or data transfer method using a memory card, as shown in FIG. 1A and FIG. 1B. Also, the communication circuit 35 transmits various exercise assistance information generated by the simulation processing in the information terminal 30 to the wrist coordinator 20, using the communication method or the transfer method described above, as shown in FIG. 1A and FIG. 1B.

The CPU 36 runs a predetermined control program based on a basic clock generated in the clock circuit 37 and thereby controls the respective operations of the display section 32, the memory 33, the training database 34, and the communication circuit 35. The clock circuit 37, which includes an oscillator for generating a basic clock, generates an operation clock that regulates the operation timing of each component of the information terminal 30, based on the basic clock.

As the operation power supply 38 in the laptop-type or tablet-type personal computer, a secondary battery such as a lithium ion battery or a commercial alternating current power supply is used. In the desktop-type personal computer, the commercial alternating current power supply is used.

### (Required Time Calculating Method)

Next, a required time calculating method in the above-described required time calculating system will be described.

The required time calculating method in the required time calculating system according to the present embodiment mainly includes a database constructing method that is a step of creating a database related to running by the user US during training, a simulation method that is a step of calculating running time for a competition course, and a required time calculating method that is a step of providing information related to running to the user US during the competition.

In the database constructing method, various training data acquired by the chest sensor 10 and the wrist coordinator 20 of the user US during training before a competition such as a marathon are collected and stored in a predetermined storage format, whereby the training database 34 is constructed. In the simulation method, based on the topography of a course where a competition such as a marathon is held, training data including the data of a similar (or matching) course is extracted from the training database 34, and simulation processing for calculating predicted time for the competition is performed. In the required time calculating method, various exercise assistance information including predicted time calculated in the simulation processing are transmitted to the wrist coordinator 20, whereby exercise assistance is provided during the competition.

These methods at each stage of the required time calculating method will be concretely described hereinafter.

### (Database Constructing Method)

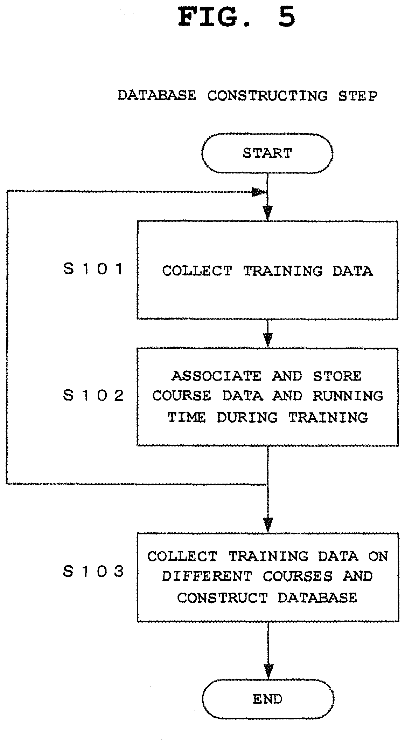

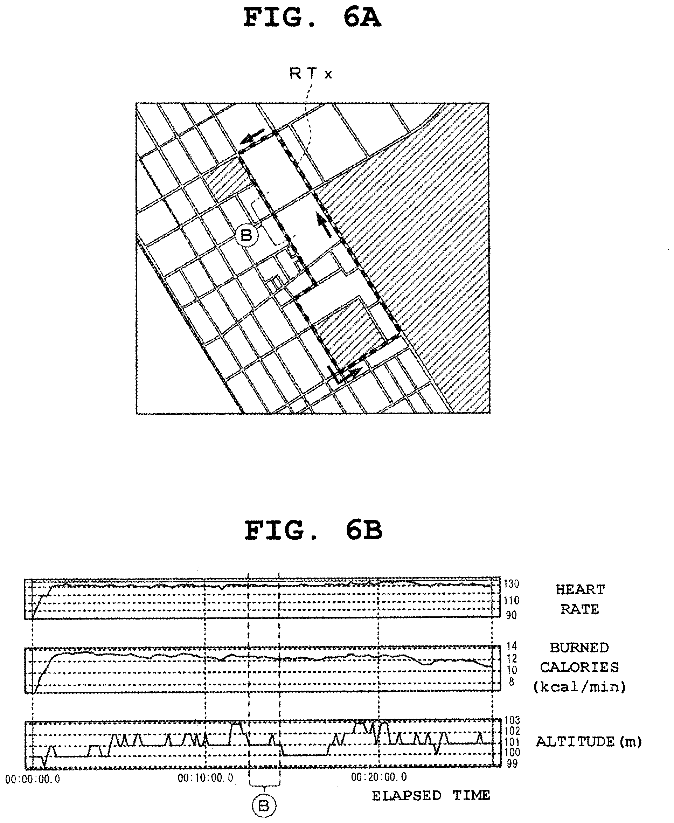

FIG. 5 is a flowchart of the database constructing method in the required time calculating method according to the present embodiment. Also, FIG. 6A and FIG. 6B are schematic diagrams showing examples of training data collected in the database constructing method according to the present embodiment. Note that the explanation herein will be made with reference to the structures shown in FIG. 1 to FIG. 4.

In the database constructing method adopted in the present embodiment, first, various training data indicating the biological information and exercise conditions of the user US during training are collected (S101), as shown in the flowchart in FIG. 5. Specifically, heart rate data, acceleration data, and angular velocity data during the training are collected from the chest sensor 10 attached to the user US as shown in FIG. 1A and FIG. 2, and stored in the memory 15 in association with time data. These heart rate data, acceleration data, and angular velocity data are transmitted to the wrist coordinator 20 constantly or at a certain time interval by, for example, a wireless communication method via the communication circuit 16. On the other hand, GPS data composed of positional (latitude and longitude) data, altitude data, movement speed data, and the like during the training is collected from the wrist coordinator 20 attached to the user US as shown in FIG. 1A and FIG. 3, and stored in the memory 24 in association with time data.

That is, training data to be collected in the processing at S101 are created by, when the user US runs a predetermined training course (measured route) RTx such as that shown in FIG. 6A toward the directions indicated by the arrows, values indicating the heart rate, burned calories, altitude, and the like being associated with time data (elapsed time) as shown in FIG. 6B. Then, these collected training data are displayed in a graph. Note that heart rate data, acceleration data, angular velocity data, and GPS data collected during the training, and various information calculated based on these data are displayed in an arbitrary format on the display panel of the wrist coordinator 20.

Then, after the training is completed, these collected training data are transmitted to the information terminal 30 such as a personal computer by a wireless communication method, a wired communication method, or a data transfer method using a memory card, via the communication circuit 25, and temporarily stored in the memory 33 of the information terminal 30 in association with time data.

Next, the data of the training course which is composed of the latitude and longitude information and the altitude information included in the GPS data in the collected training data, and the user's running time during the training are associated with each other and stored in the training database 34, as shown in FIG. 1B and FIG. 4 (S102).

As a result of the series of processing at S101 and S102 being repeatedly performed for training courses having different topographic conditions, the user's running times are associated with the courses having a variety of topographies (particularly altitude differences) and stored in the training database 34 (S103). Note that the information associated with course data during training is not limited to running time during the training. For example, training data regarding heart rate and burned calories may be associated therewith, in addition to running time. In addition, weather information regarding the temperature, humidity, and wind direction during training, and subjective information (such as physical condition, fatigue level, and life event) of the user US during training may be included in the above-described training data and associated. These pieces of information may be directly inputted by the user US via the input operating section 31 of the information terminal 30. Regarding the weather information, for example, local weather information published on the Internet may be downloaded and associated.

The training database 34 constructed by the above-described database constructing method may include a course data storage area where a training course and course data composed of the latitude and longitude and the altitude of the training course are associated with each other and stored in training course units, and a running time storage area where a training course and running time for the training course are associated with each other and stored in training course units. By these storage areas being associated in course units, corresponding running time is extracted based on the altitude difference (changes in altitude) of a course.

### (Simulation Method)

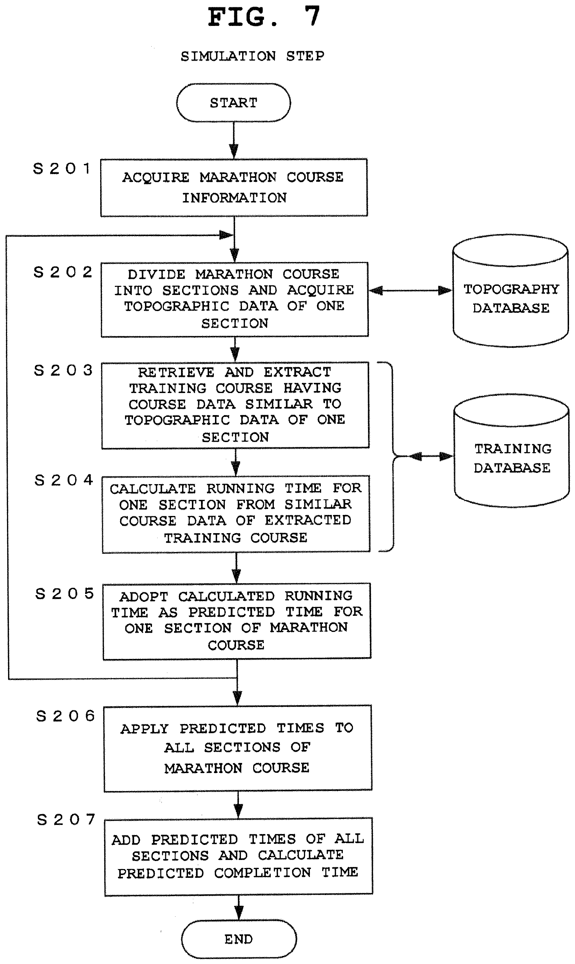

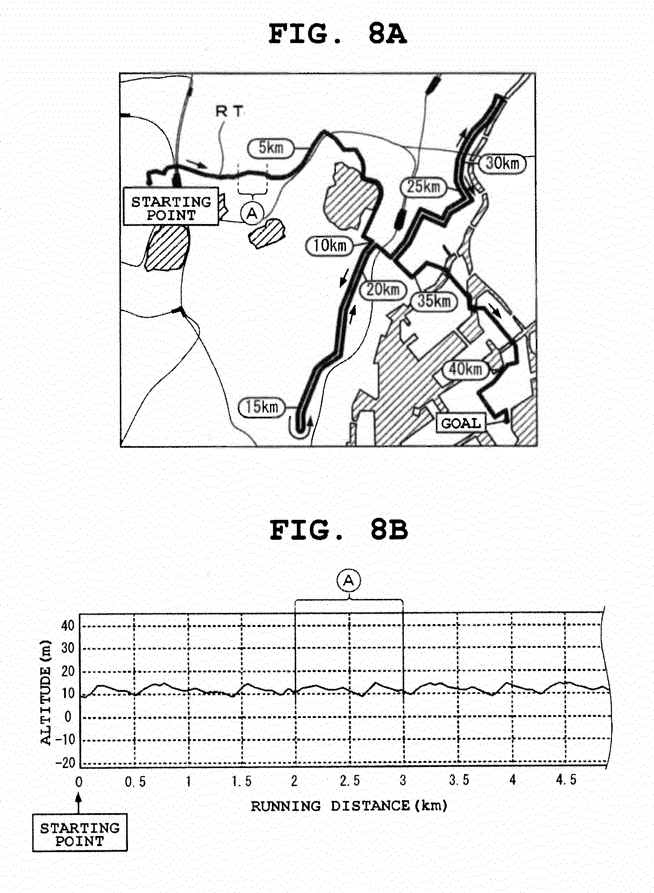

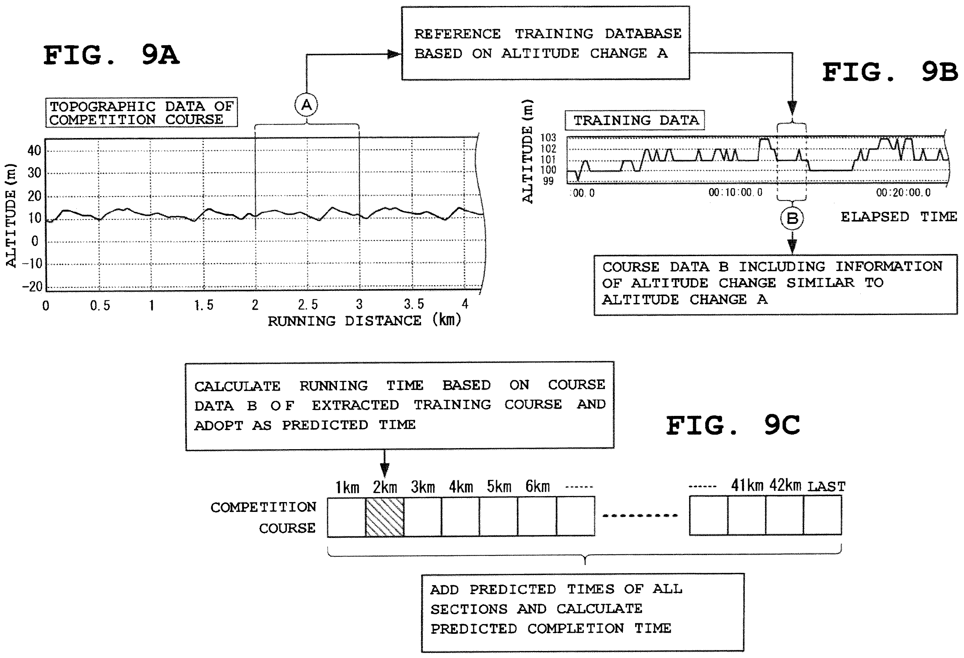

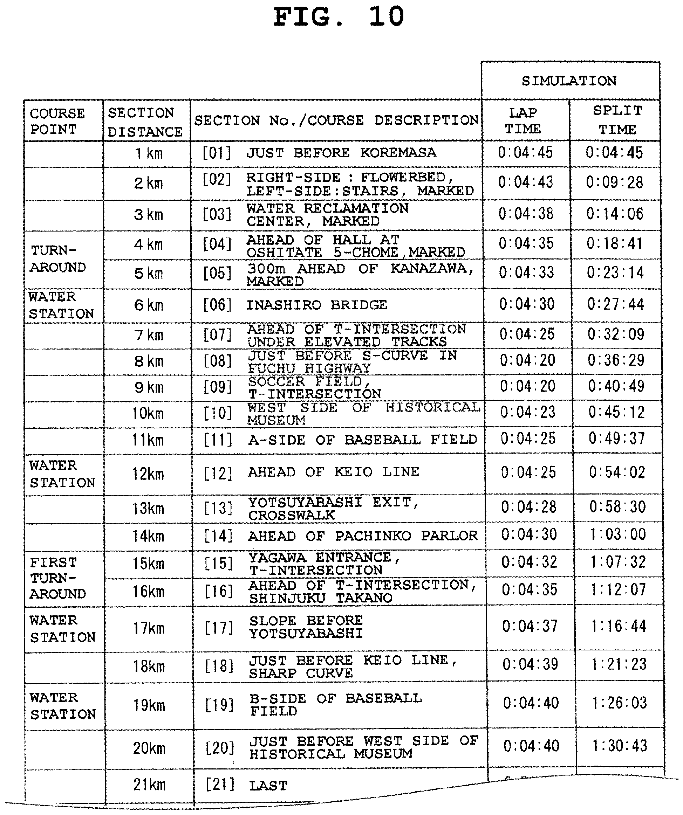

FIG. 7 is a flowchart of the simulation method in the required time calculating method according to the present embodiment, and FIG. 8A and FIG. 8B are schematic diagrams showing an example of a competition course and topographic data thereof in the simulation method according to the present embodiment. Also, FIG. 9A to FIG. 9C are conceptual diagrams showing a method for calculating predicted time in the simulation method according to the present embodiment, FIG. 10 is a diagram showing an example of exercise assistance information generated the simulation method according to the present embodiment. Note that the CPU 36 of the information terminal 30 functions as a required time calculating section, as described hereafter.

In the simulation method adopted in the present embodiment, first, the course information of a marathon event or the like where the user US is participating is acquired (S201), as shown in the flowchart in FIG. 7. Specifically, course information published on a website on a network 40, such as the Internet, by the organizer or the like of the marathon, or course information provided by the organizer or the like in the form of printed material, a Digital Versatile Disc (DVD) (formerly a Digital Video Disc), or the like is loaded by the information terminal 30.

Next, the acquired competition course of the marathon is divided into a plurality of sections per unit distance, and topographic data of one section is acquired (S202). Specifically, because normal course information provided by the organizer or the like is merely a competition course RT of a marathon or the like superimposed and displayed on a map as shown in FIG. 8A, first, the user US divides the competition course RT into a plurality of sections per unit distance, such as 1km, based on the acquired course information. Then, using the information terminal 30, the user US plots the trajectory of the course for each section in a topography database on a map site provided on the network 40 such as the Internet, or on a map provided in the form of a DVD or the like, and thereby acquires data (topographic data) related to the latitude and longitude and the altitude of one section. Note that, as the map site that provides topographic data, the Google Maps service provided on the Internet by the U.S. software company, Google (registered trademark) Inc., may be used.

That is, in the processing at S202, the altitude of an arbitrary area (an area located at an arbitrary distance from the starting point) on the course defined based on the latitude and longitude is acquired as a numerical value, and topographic data in the form of a graph is displayed based on the acquired numerical value, as shown in FIG. 8B. Note that, in a case where data related to the latitude and longitude and the altitude of the entire course is included in the course information provided by the organizer or the like, the topographic data in the form of a graph can be displayed based on the data.

Here, the unit distance when the competition course is divided into a plurality of sections is set to an arbitrary distance, such as 1km or 5km. The shorter the unit distance is, the more accurately running time (predicted time) can be calculated in the simulation of the competition. Note that, in a case where the unit distance is set to be extremely short in comparison with the entire length of the competition course (such as one-several hundredths), the simulation processing of the present embodiment may become complicated. On the other hand, in a case where the unit distance is set to, for example, about one-third of the entire length of the competition course, the accuracy of running time (predicted time) calculated in the simulation processing may be low. In the present embodiment, the unit distance is set to about 1km for a full marathon of 42.195km or a half-marathon that is half the full marathon, for example.

Next, a training course having course data similar to (or matching) the topographic data of the above-described section of the competition course is retrieved from the training database 34 (S203). Specifically, altitude change in the above-described section is extracted from the numerical value and the topographic data in the form of a graph acquired for the above-described section of the competition course. Then, a training course that has course data including information of altitude change similar to the extracted altitude change is retrieved and extracted with reference to the course data storage area of the training database 34.

That is, in the processing at S203, altitude change A in a certain section (such as a section from 2km to 3km) of the competition course is extracted from the acquired topographic data, as shown in FIG. 8A, FIG. 8B and FIG. 9A, and a training course that has course data B including information of altitude change similar to the altitude change A is extracted by the course data storage area of the training database 34 being referenced based on the altitude change A, as shown in FIG. 6A, FIG. 6B, and FIG. 9B.

Here, when course data including similar altitude change information is plurally present, only the training course that has the course data including the most similar altitude change information may be extracted. Alternatively, a plurality of training courses each having course data having a certain degree of similarity (such as course data whose information regarding the trend of altitude change is similar, course data whose information regarding the maximum altitude and the minimum altitude is similar within a certain range, course data whose information regarding the maximum altitude or the minimum altitude is similar, or course data whose information regarding the maximum altitude and the minimum altitude is similar) may be extracted. Also, when course data having a similar or matching altitude change is plurally present, a training course whose running section is most similar to the certain section (the above-described section from 2km to 3km) of the competition course may be extracted from among the plurality of training courses. That is, the topographic data of a running section whose route from the starting point of the training course to its starting point or end point is most similar to the route from the starting point of the competition course to the starting point or the end point of the certain section of the competition course may be extracted.

Next, running time for the above-described section is calculated from the course data of the extracted training course which is similar to the topographic data (S204). Specifically, the running time storage area of the training database 34 is referenced, and time data (elapsed time) associated with the course data of the extracted training course which includes information of altitude change similar to the altitude change of the above-described section of the competition course is extracted. Then, using the extracted time data, running time during training is calculated.

Here, when the running distance of the training is shorter than the entire length of the competition course, such as when training over a distance of about 5km to 10km has been repeated for a half marathon whose course distance is 21.0975km, only the course data and the running time for a distance up to 10km has been stored in the training database 34. Accordingly, when running time corresponding to the topographic data of a certain section past 10km (such as a 17km section) in the competition is calculated, running time that sufficiently reflects the user's fatigue level and characteristics during running in the certain section may not be calculated from the course data and the running time stored in the training database 34.

Therefore, in the present embodiment, a predetermined supplementation processing (or correction processing) that takes into account elements, such as the distance from the starting point of the competition course, the user's fatigue level and characteristics during running, and the like is performed on the running time of the training calculated from the course data similar only to the altitude change of the above-described section. That is, the supplementation processing is performed based on the athletic abilities of the user US at each location on the competition course.

In the present embodiment, various supplementation methods can be used. For example, a method can be used in which running time per distance is calculated taking into consideration the trend of changes in fatigue level and running time during a competition such as a marathon, using the above-described calculation chart based on VDOT proposed by Jack Daniels. Also, the following method may be used as another supplementation method. First, a number of users are sampled for each of a plurality of different categories, such as weight (body type), age, and sex, and training data in which their running distances have been associated with their running times are collected. Next, by the collected training data being statistically processed, general supplementary data or a supplementary coefficient is acquired for each category. Then, from among the acquired supplementary data and supplementary coefficients, a numerical value related to a category to which the user US who is actually calculating his or her running time belongs is applied to the above-described calculated running time. By these supplementation methods, running time that takes into account elements such as the user's fatigue level and characteristics during running can be calculated for an unknown distance (which the user US has not experienced).

Next, the running time calculated for the above-described section of the competition course is adopted as predicted time (S205). Note that, when training courses having course data similar to the topographic data of the above-described section is plurally present, the fastest running time among a plurality of running times calculated for the topographic data is applied as the predicted time, as described above. Then, the series of processing described at S202 to S205 are repeatedly performed for each section of the competition course, whereby predicted times based on the training data are applied to all the sections of the competition course (S206).

That is, in the processing at S204 to S205, the running time storage area of the training database 34 is referenced for a training course that has course data B including information of altitude change similar to the altitude change A of a certain section of a competition course, and time data associated with the course data B is extracted. Next, based on the extracted time data, calculation and the above-described supplementation processing are performed to obtain running time, and the running time is adopted as predicted time for the certain section of the competition course. Then, this calculation of predicted time is performed for all the sections of the competition course, as shown in FIG. 9C.

Next, the predicted times (running times) for the respective sections of the competition course are added and thereby the total time is calculated (S207). This total time is equivalent to predicted completion time for running all the sections of the competition course (predicted running-completion time). Here, from among the running times calculated based on course data similar to the topographic data of each section, the fastest running times are used as predicted times for each section of the competition course. Accordingly, the predicted time and the predicted running-completion time are equivalent to target sectional time and target completion time for the competition, respectively.

The predicted time for each section calculated as described above and the cumulative predicted time from the start of the competition calculated based on the predicted times are associated with various course information (such as course description and course points) acquired for the competition, and displayed on the display section 32 of the information terminal 30 as simulation results in the form of, for example, a table, as shown in FIG. 10. In FIG. 10, the predicted time for each section is expressed as "lap time", and the cumulative predicted time from the start of the competition is expressed as "split time". The "lap time" and the "split time" are terms commonly used in competitions such as marathons.

In the present embodiment, the above-described processing at S202 to S205 are repeatedly performed for each section of the competition course. However, a configuration may be adopted in which each processing is performed on all the sections of the competition course collectively. That is, a configuration may be adopted in which the processing for successively acquiring topographic data of all the sections of a competition course (S202) is performed, the processing for extracting a training course including similar topographic data (S203) is performed on all the sections, running time for each section is calculated (S204), and the processing for adopting the calculated running times as predicted times for the sections of the competition course (S205) is performed.

### (Required time calculating method)

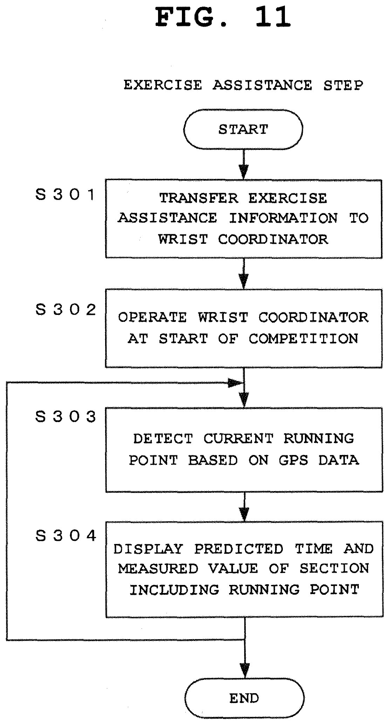

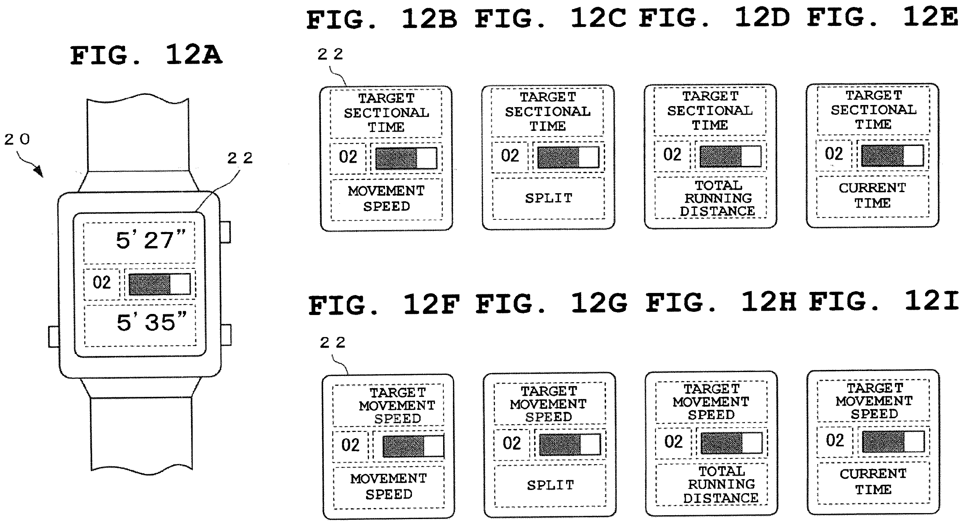

FIG. 11 is a flowchart of the required time calculating method in the required time calculating method according to the present embodiment. FIG. 12A to FIG. 12I are schematic diagrams showing display examples of exercise assistance information provided to the user during a competition by the required time calculating method according to the present embodiment.

In the required time calculating method adopted in the present embodiment, first, some or all of simulation results acquired by the above-described simulation method are transferred to the wrist coordinator 20 as exercise assistance information (S301). Specifically, among exercise assistance information such as those shown in FIG. 10, at least predicted time for each section is transferred from the information terminal 30 to the wrist coordinator 20 and stored in the memory 24.

Next, on the day of the competition where the user US is participating, the user US attaches the wrist coordinator 20 and operates the operation switches 23 of the wrist coordinator 20 simultaneously with the start of the competition (S302). As a result, exercise assistance operation in the wrist coordinator 20 is started.

Next, based on GPS data (positional information composed of latitude and longitude) acquired by the GPS reception circuit 21 included in the wrist coordinator 20, the current running point of the user US is detected (S303).

Next, predicted time for the section including the detected current running point of the user US is read out from the memory 24 and displayed in a predetermined display area of the display section 22 (S304). Note that predicted time for each section is target time for the user US in each section, as described above. The display section 22 also simultaneously displays measured section-running-time, cumulative running time, a total running distance, and the like based on the current point and the movement speed of the user US detected by the GPS reception circuit 21, in addition to the above-described predicted time (target time). That is, numerical values based on these measured values are included in the exercise assistance information, with the above-described simulation results.

As a result of the series of processing at S303 and S304 being repeatedly performed based on the current running point of the user US during the competition, the user US can perform action (such as adjusting movement speed) required to achieve the target completion time by checking exercise assistance information composed of simulation results and various measured values displayed on the wrist coordinator 20.

Here, display examples of the display section 22 of the wrist coordinator 20 will be described. For example, in the wristwatch-type wrist coordinator 20 shown in FIG. 12A, predicted time (section target time) for the section including the current running point of the user US is displayed in the upper row of the display area of the display section 22 in the drawing. Also, in the lower row of the display area in the drawing, section running time based on a measured value is displayed. On the left side of the middle row of the display area of the display section 22 in the drawing, the section number of the section including the current running point of the user US is displayed. On the right side of the middle row of the display area, the current running distance in the section is displayed by an indicator. Note that the display herein may be numerical values, indicators, graphs, etc. Also note that the push buttons provided on side portions of the wrist coordinator 20 in FIG. 12A are the operation switches 23, such as the above-described mode switching switch and display switching switch, a switch for measuring running time for each section, and a screen lock switch for a structure where a touch panel has been adopted as the display section 22.

In the required time calculating method of the present embodiment, the contents or the type of measured values displayed in the lower row of the display area of the display section 22 in the drawing can be changed by the operation switches 23 or the touch panel of the display section 22 of the wrist coordinator 20 being operated, as shown in FIG. 12B to FIG. 12E. For example, FIG. 12B shows a state where the current movement speed (pace) of the user US is being displayed. Also, FIG. 12C shows a state where the measured value of cumulative time (split time) from the start of a competition is being displayed. FIG. 12D shows a state where the measured value of a running distance from the starting point of a competition is being displayed. FIG. 12E shows the current time.

In the display examples shown in FIG. 12A to FIG. 12E, predicted time (section target time) for each section is displayed in the upper row of the display area of the display section 22 in the drawing. However, the present invention is not limited thereto. That is, a configuration may be adopted in which predicted movement speed (target movement speed) for each section is calculated in the simulation method, based on training data stored by the above-described database constructing method, and displayed in the wrist coordinator 20 as exercise assistance information. Display examples of the wrist coordinator 20 in this configuration are shown in FIG. 12F to FIG. 12I.

That is, based on the current running point, the user US in a competition compares, for example, section target time (5'27" in FIG. 12A) displayed in the upper row of the display section 22 as in FIG. 12A, and section running time (5'35" in FIG. 12A) displayed in the lower row of the display section 22 which is achieved when the current running method is continued based on a measured value, and adjusts his or her movement speed to achieve the section target time. Alternatively, the user US compares target movement speed displayed in the upper row of the display section 22 and the current movement speed based on a measured value displayed in the lower row of the display section 22 as in FIG. 12R, and adjusts his or her movement speed to achieve target movement speed.

As described above, in the present embodiment, the topography of the actual course of a competition such as a marathon and the data of a course where the user US has run in the past during training are compared, completion time and running time for a section in the actual competition are predicted based on running time during training in the similar (or matching) course data, and exercise assistance for achieving a target result is provided by the information of the predicted times being provided to the user in real-time during the actual competition.

In a common simulation method for predicting target completion time or the like for competitions such as a marathon, past running time per arbitrary unit distance is simply converted to the entire length of a competition course (such as 42.195km), or the result thereof is simply multiplied by a coefficient for fatigue level or athletic intensity. In this method, the characteristics of an actual competition course (the topography in particular) and the athletic abilities of the user when running the course are not sufficiently taken into consideration. Therefore, the accuracy and the reliability of simulation results by this method are low.

In the present invention, predicted time (target time) for each section is calculated from past training data of the user running a course that has topographic data similar to (or matching) the topographic data of the actual competition course, whereby a simulation for predicted time, in which the characteristics of the competition course and the athletic abilities of the user when running the competition course are reflected, can be performed in a condition more similar to the actual running. Accordingly, in the present invention, predicted time can be calculated from simulation results that are highly accurate and reliable, and the user can be provided with suitable exercise assistance information.

In the present embodiment, simulation results of the simulation method and various measured values are displayed as exercise assistance information on the display section 22 of the wrist coordinator 20 during a competition. However, the present invention is not limited thereto. That is, a configuration may be adopted in which, in the above-described required time calculating method, various information (such as the position of the user US, the altitude, the movement speed, and the actual running time detected by the GPS reception circuit 21) measured during an actual competition are stored in the memory 24, and then transmitted to the information terminal 30 via the communication circuit 25 and stored in the training database 34 in association with one another after the competition, as in the case of the above-described database construction method. In this configuration, the actual running time, the fatigue level, the characteristics of running, and the like of the user US during a competition can be acquired, whereby running time (predicted time) and the like can be more accurately calculated in subsequent competition simulations.

### (Variation Examples)

Next, a variation example of the required time calculating system, the required time calculating program, and the required time calculating method according to the embodiment of the present invention will be described.

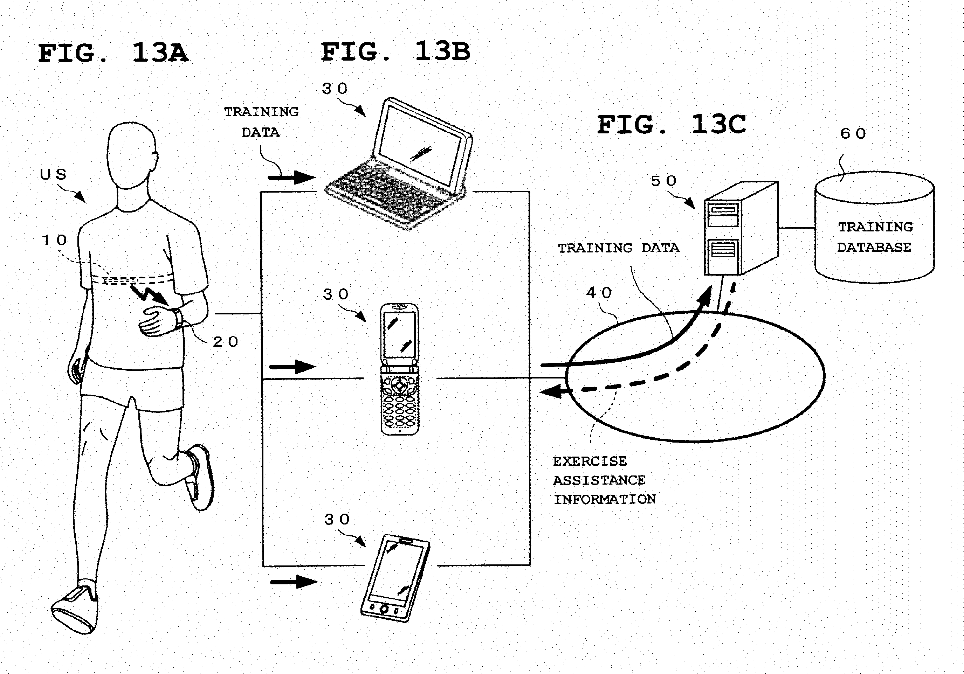

FIG. 13A to FIG. 13B are structural diagrams outlining a variation example of the required time calculating system according to the embodiment of the present invention. Note that the components thereof that are the same as those of the above-described embodiment are described using the same reference numbers.

In the above-described embodiment, a personal computer in which the training database 34 is included or to which the training database 34 is connected by a connection cable is used as the information terminal 30, and a so-called stand-alone system is provided in which the database construction method and the simulation method are performed by the information terminal 30 and exercise assistance information is transferred to and displayed on the wrist coordinator 20. However, the present invention is not limited thereto. In the present variation example, a cloud computing system is used.

That is, the required time calculating system of the present variation example includes the network 40 to which the information terminal 30 according to the above-described embodiment is connected, a server 50 connected to the network 40, and a training database 60 connected to the server 50.

As shown in FIG. 13B, a personal computer, a mobile phone, a highly functional mobile phone (so-called smart phone), a mobile information terminal (tablet or Personal Digital Assistant [PDA]), a dedicated terminal, or the like that has at least a function for connecting to the network 40 such as the Internet can be used as the information terminal 30. This information terminal 30 does not include or is not connected to the training database 34 according to the above-described embodiment. As a method for connecting the information terminal 30 to the network 40, for example, a wired connection method in which connection to the network 40 is made via a fiber optics network or an Asymmetric Digital Subscriber Line (ADSL) network, or a wireless connection method in which connection to the network 40 is made via a mobile phone network or a high-speed mobile communication network can be used.

The network 40 may be the Internet or a local network, such as a wireless Local Area Network (LAN) or a wired LAN. The server 50 is an application server including the training database 60 which is equivalent to that of the above-described embodiment, and stores various training data transmitted (uploaded) from the information terminal 30 via the network 40 in the training database 60 in a storage format similar to that of the above-described embodiment, as shown in FIG. 13C.

The server 50, which is an application server having the training database 60 and connected to the network 40 such as the Internet, includes an input operating section 51 and a display section 52 to enable operation and the viewing of information by an operator. This server 50 performs simulations using the training database 60, generates exercise assistance information during a competition, and transmits (downloads) the generated exercise assistance information to the information terminal 30 via the network 40. That is, in the present variation example, the CPU 56 of the server 50 functions as a required time calculating section. Specifically, the server 50 includes the input operating section 51, the display section 52, a memory 53, a communication circuit 55, the CPU (certain information generation processing section) 56, a clock circuit 57, an operation power supply 58, and the training database 60.

The input operating section 51 includes an input device, such as a keyboard, a mouse, a touch pad, or a touch panel. By the operation of the input device by the user US, an arbitrary icon or menu on a screen displayed on the display section 52 is selected, or an arbitrary position is specified. The display section 52 includes a display panel such as a liquid crystal display panel, or a monitor, and displays information related to various operations of the server 50.

The memory 53 mainly includes a data memory that stores various training data transmitted from the information terminal 30, and a program memory that stores control programs (software) for performing various functions of the display section 52, the memory 53, and the communication circuit 55. The CPU 56 actualizes the functions of the display section 52, the memory 53, and the communication circuit 55 by performing processing in accordance with these control programs.

The communication circuit 55 functions as an interface when various training data are transmitted between the server 50 and the information terminal 30 connected to the network 40. The communication circuit 55 receives various training data from the information terminal 30, using a wireless communication method, a wired communication method, or a data transfer method using a memory card, as shown in FIG. 13C.

The CPU 56 runs a predetermined control program based on a basic clock generated in the clock circuit 57 and thereby controls the respective operations of the display section 52, the memory 53, and the communication circuit 55. The clock circuit 57, which includes an oscillator for generating a basic clock, generates an operation clock that regulates the operation timing of each component of the server 50, based on the basic clock. As the operation power supply 58, a commercial alternating current power supply is used.

As described above, the server 50 connected to the network 40 includes the training database 60, and a device having a function for connecting to the network 40 is used as the information terminal 30. As a result, processing load on the information terminal 30, such as database control and simulation processing, can be significantly reduced.

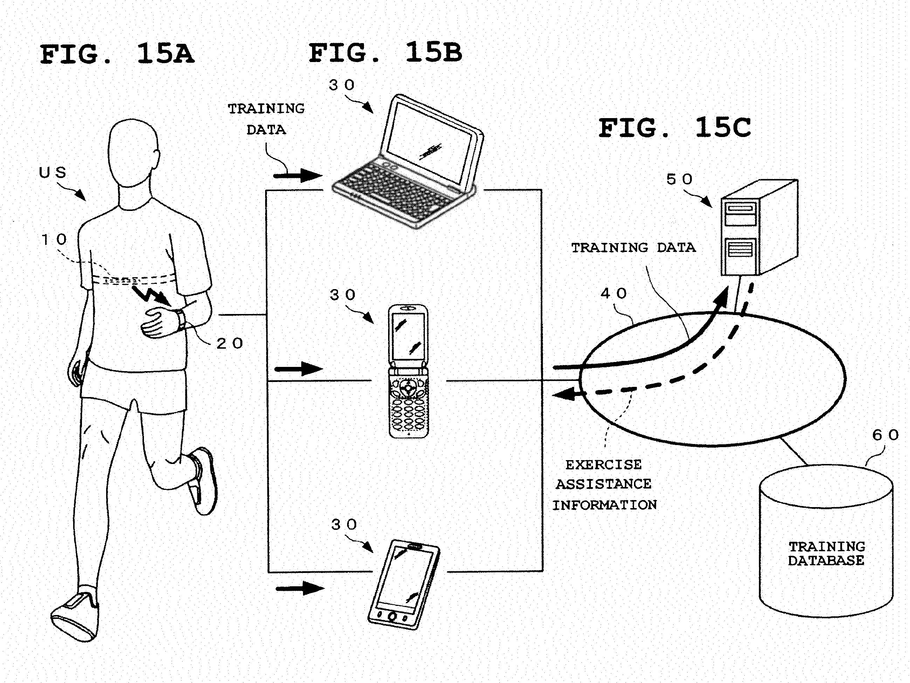

Also, in the case where a mobile phone, a smartphone, or the like is used as the information terminal 30, since they already have the function for connecting to the network 40 by a wireless connection method, acquired training data can be easily uploaded to the server 50 via the network 40 regardless of location as long as the information terminal 30 is within a communicable range, and exercise assistance information generated in the server 50 can be downloaded via the network 40 and viewed. In addition, by adopting a configuration where operations for uploading training data, downloading exercise assistance information, displaying various information, and the like can be performed on a typical web browser included in the personal computer, the mobile phone, the smartphone, and the like, the required time calculating system and the required time calculating method of the present invention can be actualized by a simple configuration using the information terminal 30, without requiring special software. Note that, as a second variation example, a configuration may be adopted in which the CPU 56 of the server 50 in the above-described variation example acquires training data from the training database 60 via the network 40 by a wired or wireless method, as shown in FIG. 15.

In the above-described embodiment and variation examples, a marathon event is given as an example of a competition to which the present invention is applied. However, the present invention is not limited thereto, and may be applied to various competitions and exercises, such as cycling races, mountain climbing, and trekking.

## Claims

1. A required time calculating system comprising:
a required time calculating section (36, 56) configured to:
store, in a training database (34), topographic data, including positional information and altitude information, in association with time data of at least one measured route;
divide a calculated route into a plurality of sections, and acquire topographic data, including positional information and altitude information, of each of the plurality of sections of the calculated route, wherein the calculated route is different from the at least one measured route;
extract, from the training database, for each of the plurality of sections of the calculated route, time data for one section of the at least one measured route associated with topographic data that is similar to or matching topographic data of the respective section of the calculated route; and
calculate an amount of time required for a human to travel the calculated route by calculating and adding amounts of time required to travel each of the plurality of sections of the calculated route, based on the extracted time data indicating amounts of time taken for the human to travel the respective sections of the at least one measured route,
wherein said topographic data is acquired by a first sensor (21) which is configured to travel with the human when the human travels the at least one measured route.

2. The required time calculating system according to claim 1, wherein the required time calculating section (36, 56) is configured to extract, when the topographic data of the one section of the measured route similar to the topographic data of the one section of the calculated route are plurally present, topographic data of the one section of the measured route whose information regarding maximum altitude or minimum altitude of the one section of the measured route is similar, within a certain range, to information regarding maximum altitude or minimum altitude of the one section of the calculated route from among the plurality of topographic data of the one section of the measured route.

3. The required time calculating system according to claim 1, wherein the required time calculating section (36, 56) is configured to extract, when the topographic data of the one section of the measured route similar to the topographic data of the one section of the calculated route are plurally present, topographic data of the one section of the measured route whose information regarding a route from a starting point of the measured route to a starting point or an end point of the one section of the measured route is within a certain range compared to information regarding a route from a starting point of the calculated route to a starting point or an end point of the one section of the calculated route from among the plurality of topographic data of the one section of the measured route.

4. The required time calculating system according to claim 1, wherein the training database includes a plurality of measured routes having different topographies; and
the required time calculating section (36, 56) is configured to extract the topographic data of the one section of the measured route similar to or matching the topographic data of the one section of the calculated route, from a plurality of topographic data acquired from the plurality of measured routes.

5. The required time calculating system according to claim 1, wherein the first sensor (21) is comprised in the required time calculating system, and is configured to move with the human, and detect the positional information and the altitude information of each point of the measured route when the human travels the one section of the measured route.

6. The required time calculating system according to claim 5, wherein the required time calculating section (36, 56) is configured to acquire the positional information and the altitude information of each point of the measured route detected by the first sensor (21), via a communication network (40).

7. The required time calculating system according to claim 1, further comprising:
a second sensor (12) and a third sensor (13) configured to move with the human and detect acceleration and angular velocity, respectively, of the human at each point of the measured route, when the human travels the one section of the measured route.

8. The required time calculating system according to claim 1, wherein, in storing, the required time calculating section (36, 56) is configured to associate the positional information and the altitude information of each point of the measured route with time at which the human has passed each point.

9. The required time calculating system according to claim 1, wherein the required time calculating section (36, 56) is configured to extract the positional information and the altitude information stored in the training database (24), via a communication network.

10. A non-transitory computer-readable storage medium having stored thereon a required time calculating program that is executable by a computer, the program being executable by the computer to perform functions comprising:
storing, in a training database (34), topographic data, including positional information and altitude information, in association with time data of at least one measured route;
dividing a calculated route into a plurality of sections, and acquiring topographic data, including positional information and altitude information, of each of the plurality of sections of the calculated route, wherein the calculated route is different from the at least one measured route;
extracting, from the training database, for each of the plurality of sections of the calculated route, time data for one section of the at least one measured route associated with topographic data that is similar to or matching topographic data of the respective section of the calculated route; and
calculating an amount of time required for a human to travel the calculated route by calculating and adding amounts of time required to travel each of the plurality of sections of the calculated route based on the extracted time data indicating amounts of time taken for the human to travel the respective sections of at least one the measured route,
wherein said topographic data is acquired by a first sensor (21) which is configured to travel with the human when the human travels the at least one measured route.

11. A required time calculating method comprising the steps of:
storing, in a training database (34), topographic data, including positional information and altitude information, in association with time data of at least one measured route;
dividing a calculated route into a plurality of sections, and acquiring topographic data, including positional information and altitude information, of each of the plurality of sections of the calculated route, wherein the calculated route is different from the at least one measured route;
extracting, from the training database, for each of the plurality of sections of the calculated route, data for one section of the at least one measured route associated with topographic data that is similar to or matching topographic data of the respective section of the calculated route; and
calculating an amount of time required for a human to travel the calculated route by calculating and adding amounts of time required to travel each of the plurality of sections of the calculated route based on the extracted time data indicating amounts of time taken for the human to travel the respective sections of the at least one measured route,
wherein said topographic data is acquired by a first sensor (21) which is configured to travel with the human when the human travels the at least one measured route.

## Patentansprüche

1. System zur Berechnung eines Zeitbedarfs, mit:
einem Abschnitt zur Berechnung eines Zeitbedarfs (36, 56), der ausgebildet ist, um:
in einer Übungsdatenbank (34) topografische Daten einschließlich von Positionsinformation und Höheninformation im Zusammenhang mit Zeitdaten mindestens einer gemessenen Route zu speichern;
eine berechnete Route in mehrere Abschnitte zu unterteilen und topografische Daten einschließlich von Positionsinformation und Höheninformation für jeden der mehreren Abschnitte der berechneten Route zu ermitteln, wobei die berechnete Route sich von der mindestens einen gemessenen Route unterscheidet;
aus der Übungsdatenbank für jeden der mehreren Abschnitte der berechneten Route Zeitdaten für einen einzelnen Abschnitt der mindestens einen gemessenen Route, die mit topografischen Daten im Zusammenhang stehen, die ähnlich oder übereinstimmend zu topografischen Daten des entsprechenden Abschnitts der berechneten Route sind, auszu lesen; und
einen Betrag der Zeit zu berechnen, der für eine Person erforderlich ist, um die berechnete Route zurückzulegen, indem Beträge von Zeiten, die zum Zurücklegen jedes der mehreren Abschnitte der berechneten Route erforderlich sind, auf der Grundlage der ausgelesenen Zeitdaten, die Beträge einer Zeit angeben, die die Person zum Zurücklegen der jeweiligen Abschnitte der mindestens einen gemessenen Route benötigte, berechnet und addiert werden,
wobei die topografischen Daten durch einen ersten Sensor (21) ermittelt werden, der ausgebildet ist, zusammen mit der Person die mindestens eine gemessene Route zurückzulegen.

2. System zur Berechnung eines Zeitbedarfs nach Anspruch 1, wobei, wenn die topografischen Daten des einen Abschnitts der gemessenen Route, die ähnlich zu den topografischen Daten des einen Abschnitts der berechneten Route sind, vielfach vorhanden sind, der Abschnitt zur Berechnung eines Zeitbedarfs (36, 56) ausgebildet ist, topografische Daten des einen Abschnitts der gemessenen Route, dessen Information bezüglich maximaler Höhe oder minimaler Höhe des einen Abschnitts der gemessenen Route ähnlich, innerhalb eines gewissen Bereichs, zur Information bezüglich maximaler Höhe oder minimaler Höhe des einen Abschnitts der berechneten Route ist, aus den mehreren topografischen Daten des einen Abschnitts der gemessenen Route zu extrahieren.

3. System zur Berechnung eines Zeitbedarfs nach Anspruch 1, wobei, wenn die topografischen Daten des einen Abschnitts der gemessenen Route, die ähnlich zu den topografischen Daten des einen Abschnitts der berechneten Route sind, vielfach vorhanden sind, der Abschnitt zur Berechnung eines Zeitbedarfs (36, 56) ausgebildet ist, topografische Daten des einen Abschnitts der gemessenen Route, dessen Information bezüglich einer Route von einem Anfangspunkt der gemessenen Route zu einem Anfangspunkt oder einem Endpunkt des einen Abschnitts der gemessenen Route innerhalb eines gewissen Bereichs im Vergleich zur Information bezüglich einer Route von einem Anfangspunkt der berechneten Route zu einem Anfangspunkt oder einem Endpunkt des einen Abschnitts der berechneten Route liegt, aus den mehreren topografischen Daten des einen Abschnitts der gemessenen Route zu extrahieren.

4. System zur Berechnung eines Zeitbedarfs nach Anspruch 1, wobei die Übungsdatenbank mehrere gemessene Routen mit unterschiedlichen Topografien enthält; und
der Abschnitt zur Berechnung eines Zeitbedarfs (36, 56) ausgebildet ist, die topografischen Daten des einen Abschnitts der gemessenen Route, die ähnlich sind zu oder mit den topografischen Daten des einen Abschnitts der berechneten Route übereinstimmen, aus mehreren topografischen Daten, die aus den mehreren gemessenen Routen ermittelt sind, zu extrahieren.

5. System zur Berechnung eines Zeitbedarfs nach Anspruch 1, wobei der erste Sensor (21) in dem System zur Berechnung eines Zeitbedarfs enthalten und ausgebildet ist, sich zusammen mit der Person zu bewegen und die Positionsinformation und die Höheninformation jedes Punktes der gemessenen Route zu erfassen, wenn die Person den einen Abschnitt der gemessenen Route zurücklegt.

6. System zur Berechnung eines Zeitbedarfs nach Anspruch 5, wobei der Abschnitt zur Berechnung eines Zeitbedarfs (36, 56) ausgebildet ist, die Positionsinformation und die Höheninformation jedes Punktes der gemessenen Route, der von dem ersten Sensor (21) erfasst wird, über ein Kommunikationsnetzwerk (40) zu ermitteln.

7. System zur Berechnung eines Zeitbedarfs nach Anspruch 1, das ferner umfasst:
einen zweiten Sensor (12) und einen dritten Sensor (13), die ausgebildet sind, sich mit der Person zu bewegen und entsprechend Beschleunigung und Winkelgeschwindigkeit der Person an jedem Punkt der gemessenen Route zu erfassen, wenn die Person den einen Abschnitt der gemessenen Route zurücklegt.

8. System zur Berechnung eines Zeitbedarfs nach Anspruch 1, wobei beim Speichern der Abschnitt zur Berechnung eines Zeitbedarfs (36, 35) ausgebildet ist, die Positionsinformation und die Höheninformation eines jeweiligen Punktes der gemessenen Route einer Zeit zuzuordnen, an der die Person den jeweiligen Punkt passiert hat.

9. System zur Berechnung eines Zeitbedarfs nach Anspruch 1, wobei der Abschnitt zur Berechnung eines Zeitbedarfs (36, 56) ausgebildet ist, die Positionsinformation und die Höheninformation, die in der Übungsdatenbank (24) gespeichert sind, über ein Kommunikationsnetzwerk auszulesen.

10. Nicht-flüchtiges computerlesbares Speichermedium mit einem Programm zur Berechnung eines Zeitbedarfs, das darauf gespeichert und von einem Computer ausführbar ist, wobei das Programm von dem Computer ausführbar ist, um Funktionen auszuführen, die umfassen:
Speichern von topografischen Daten in einer Übungsdatenbank (34), die Positionsinformation und Höheninformation enthalten, unter Zuordnung von Zeitdaten für mindestens eine gemessene Route;
Unterteilen einer berechneten Route in mehrere Abschnitte und Ermitteln von topografischen Daten einschließlich von Positionsinformation und Höheninformation für jeden der mehreren Abschnitte der berechneten Route, wobei die berechnete Route sich von der mindestens einen gemessenen Route unterscheidet;
Auslesen, aus der Übungsdatenbank für jeden der mehreren Abschnitte der berechneten Route, von Zeitdaten für einen Abschnitt der mindestens einen gemessenen Route, die topografischen Daten zugeordnet sind, die ähnlich sind zu den topografischen Daten des entsprechenden Abschnitts der berechneten Route oder damit übereinstimmen; und
Berechnen eines Betrags an Zeit, der für eine Person erforderlich ist, um die berechnete Route zurückzulegen, indem Beträge von Zeiten, die zum Zurücklegen jedes der mehreren Abschnitte der berechneten Route erforderlich sind, auf der Grundlage der ausgelesenen Zeitdaten, die Beträge der Zeit angeben, die für die Person zum Zurücklegen der jeweiligen Abschnitte der mindestens einen gemessenen Route erforderlich sind, berechnet und addiert werden,
wobei die topografischen Daten von einem ersten Sensor (21) ermittelt werden, der ausgebildet ist, die mindestens eine gemessene Route zusammen mit der Person zurückzulegen.

11. Verfahren zur Berechnung eines Zeitbedarfs mit den Schritten:
Speichern von topografischen Daten einschließlich von Positionsinformation und Höheninformation in einer Übungsdatenbank (34) unter Zuordnung von Zeitdaten für mindestens eine gemessene Route;
Unterteilen einer berechneten Route in mehrere Abschnitte und Ermitteln von topografischen Daten einschließlich von Positionsinformation und Höheninformation für jeden der mehreren Abschnitte der berechneten Route, wobei die berechnete Route sich von der mindestens einen gemessenen Route unterscheidet;
Auslesen, aus der Übungsdatenbank für jeden der mehreren Abschnitte der berechneten Route, von Daten für einen Abschnitt der mindestens einen gemessenen Route, die topografischen Daten zugeordnet sind, die ähnlich zu oder übereinstimmend mit den topografischen Daten des entsprechenden Abschnitts der berechneten Route sind; und
Berechnen eines Betrags an Zeit, der für eine Person erforderlich ist, die berechnete Route zurückzulegen, indem Beträge von Zeiten, die zum Zurücklegen jedes der mehreren Abschnitte der berechneten Route erforderlich sind, auf der Grundlage der ausgelesenen Zeitdaten, die Beträge von Zeiten angeben, die für die Person zum Zurücklegen der entsprechenden Abschnitte der mindestens einen gemessenen Route erforderlich sind, berechnet und addiert werden,
wobei die topografischen Daten von einem ersten Sensor (21) ermittelt werden, der ausgebildet ist, die mindestens eine gemessene Route zusammen mit der Person zurückzulegen.

## Revendications

1. Système de calcul de temps requis comprenant :
une section de calcul de temps requis (36, 56) configurée pour :
mémoriser, dans une base de données d'apprentissage (34) des données topographiques incluant des informations de position et des informations d'altitude en association avec des données de temps d'au moins un chemin mesuré,
diviser un chemin calculé en une pluralité de sections et acquérir des données topographiques incluant des informations de position et des informations d'altitude sur chacune de la pluralité de sections du chemin calculé, le chemin calculé étant différent du ou des chemins mesurés,
extraire de la base de données d'apprentissage, pour chacune de la pluralité de sections du chemin calculé, des données de temps pour une section du ou des chemins mesurés associées à des données topographiques qui sont semblables ou qui correspondent aux données topographiques de la section respective du chemin calculé, et
calculer la quantité de temps requise pour qu'un être humain parcoure le chemin calculé en calculant et en ajoutant des quantités de temps requises pour parcourir chacune de la pluralité de sections du chemin calculé sur la base des données de temps extraites indiquant des quantités de temps prises pour que l'être humain parcoure les sections respectives du ou des chemins mesurés,
dans lequel lesdites données topographiques sont acquises par un premier capteur (21) qui est configuré pour voyager avec l'être humain lorsque l'être humain parcourt le ou les chemins mesurés.

2. Système de calcul de temps requis selon la revendication 1, dans lequel la section de calcul de temps requis (36, 56) est configurée pour extraire, lorsque les données topographiques de ladite section du chemin mesuré semblables aux données topographiques de ladite section du chemin calculé sont présentes collectivement, les données topographiques de ladite section du chemin mesuré dont les informations se rapportant à l'altitude maximale où à l'altitude minimale de ladite section du chemin mesuré sont semblables, dans une certaine plage, à des informations se rapportant à l'altitude maximale où à l'altitude minimale de ladite section du chemin calculé parmi la pluralité de topographiques de ladite section du chemin mesuré.

3. Système de calcul de temps requis selon la revendication 1, dans lequel la section de calcul de temps requis (36, 56) est configurée pour extraire, lorsque les données topographiques de ladite section des chemins mesurés semblables aux données topographiques de ladite section du chemin calculé sont présentes collectivement, les données topographiques de ladite section du chemin mesuré dont les informations se rapportant à un chemin depuis un point de départ du chemin mesuré jusqu'à un point de départ ou un point d'arrivée de ladite section du chemin mesuré se trouvent dans une certaine plage par comparaison aux informations se rapportant à un chemin allant d'un point de départ du chemin calculé jusqu'à un point de départ ou un point d'arrivée de ladite section du chemin calculé parmi la pluralité de données topographiques de ladite section du chemin mesuré.

4. Système de calcul de temps requis selon la revendication 1, dans lequel la base de données d'apprentissage inclut une pluralité de chemins mesurés présentant des topographiques différentes, et
la section de calcul de temps requis (36, 56) est configurée pour extraire les données topographiques de ladite section du chemin mesuré qui sont semblables ou qui correspondent aux données topographiques de ladite section du chemin calculé à partir de pluralité de données topographiques acquises à partir de la pluralité de chemins mesurés.

5. Système de calcul de temps requis selon la revendication 1, dans lequel le premier capteur (21) est compris dans le système de calcul de temps requis et il est configuré pour se déplacer avec l'être humain et pour détecter les informations de position et les informations d'altitude de chaque point du chemin mesuré lorsque l'être humain parcourt ladite section du chemin mesuré.

6. Système de calcul de temps requis selon la revendication 5, dans lequel la section de calcul de temps requis (36, 56) est configurée pour acquérir les informations de position et les informations d'altitude de chaque point du chemin mesuré détecté par le premier capteur (21) par l'intermédiaire d'un réseau de communication (40).

7. Système de calcul de temps requis selon la revendication 1, comprenant en outre :
un deuxième capteur (12) et un troisième capteur (13) configurés pour se déplacer avec l'être humain et pour détecter respectivement l'accélération et la vitesse angulaire de l'être humain à chaque point du chemin mesuré lorsque l'être humain parcourt ladite section du chemin mesuré.

8. Système de calcul de temps requis selon la revendication 1, dans lequel, lors de la mémorisation, la section de calcul de temps requis (36, 56) est configurée pour associer les informations de position et les informations d'altitude de chaque point du chemin mesuré avec l'instant auquel l'être humain a passé chaque point.

9. Système de calcul de temps requis selon la revendication 1, dans lequel la section de calcul de temps requis (36, 56) est configurée pour extraire les informations de position et les informations d'altitude mémorisées dans la base de données d'apprentissage (24) par l'intermédiaire d'un réseau de communication.

10. Support de stockage non transitoire pouvant être lu par ordinateur comportant, stocké, un programme de calcul de temps requis exécutable par un ordinateur, le programme étant exécutable par l'ordinateur pour réaliser des fonctions comprenant :
la mémorisation, dans une base de données d'apprentissage (34), de données topographiques incluant des informations de position et des informations d'altitude en association avec des données de temps d'au moins un chemin mesuré,
la division d'un chemin calculé en une pluralité de sections et l'acquisition de données topographiques incluant des informations de position et des informations d'altitude sur chacune de la pluralité de sections du chemin calculé, le chemin calculé étant différent du ou des chemins mesurés,
l'extraction, de la base de données d'apprentissage, pour chacune de la pluralité de sections du chemin calculé, de données de temps pour une section du ou des chemins mesurés associées à des données topographiques qui sont semblables ou qui correspondent aux données topographiques de la section respective du chemin calculé, et
le calcul de la quantité de temps requise pour qu'un être humain parcoure le chemin calculé en calculant et en ajoutant des quantités de temps requises pour parcourir chacune de la pluralité de sections du chemin calculé sur la base des données de temps extraites indiquant des quantités de temps prises pour que l'être humain parcoure les sections respectives du ou des chemins mesurés,
dans lequel lesdites données topographiques sont acquises par un premier capteur (21) qui est configuré pour voyager avec l'être humain lorsque l'être humain parcourt le ou les chemins mesurés.

11. Procédé de calcul de temps requis comprenant les étapes suivantes :
la mémorisation, dans une base de données d'apprentissage (34), de données topographiques incluant des informations de position et des informations d'altitude en association avec , des données de temps d'au moins un chemin mesuré,
la division d'un chemin calculé en une pluralité de sections et l'acquisition de données topographiques incluant des informations de position et des informations d'altitude sur chacune de la pluralité de sections du chemin calculé, le chemin calculé étant différent du ou des chemins mesurés,
l'extraction, de la base de données d'apprentissage, pour chacune de la pluralité de sections du chemin calculé, de données de temps pour une section du ou des chemins mesurés associées à des données topographiques qui sont semblables ou qui correspondent aux données topographiques de la section respective du chemin calculé, et
le calcul de la quantité de temps requise pour qu'un être humain parcoure le chemin calculé en calculant et en ajoutant des quantités de temps requises pour parcourir chacune de la pluralité de sections du chemin calculé sur la base des données de temps extraites indiquant des quantités de temps prises pour que l'être humain parcoure les sections respectives du ou des chemins mesurés,
dans lequel lesdites données topographiques sont acquises par un premier capteur (21) qui est configuré pour voyager avec l'être humain lorsque l'être humain parcourt le ou les chemins mesurés.
